# EUROPEAN PATENT SPECIFICATION

(11) **EP 4 505 878 B1**
(45) Date of publication and mention of the grant of the patent: **09.09.2026**
(21) Application number: 23715897.7
(22) Date of filing: 29.03.2023
(51) Int. Cl.: A23B 2/00, A23B 2/46, A23B 70/30

(54) **METHOD OF REDUCTION AND/OR ELIMINATION OF A TARGET AGENT**
VERFAHREN ZUR REDUKTION UND/ODER ELIMINIERUNG EINES ZIELMITTELS
PROCÉDÉ DE RÉDUCTION ET/OU D'ÉLIMINATION D'AGENT CIBLE

(30) Priority: 01.04.2022 ES 202230297
(43) Date of publication of application: 12.02.2025
(73) Proprietor: GEMINA I MAS D S.L., 30520 Jumilla (ES)
(72) Inventor: SIMÓN BERNAL, Pedro, 30520 Jumilla (Murcia) (ES); SIMÓN GARCÍA, Pedro José, 30520 Jumilla (Murcia) (ES); VILELLA ESPLÁ, José, 30520 Jumilla (Murcia) (ES)
(74) Representative: Ungria López, Javier
(86) International application number: PCT/ES2023/070204
(87) International publication number: WO 2023/187240

(56) References cited:
- EP-A1- 1 106 083
- EP-B1- 2 935 056
- WO-A1-2008/132046
- CN-A- 110 531 799
- US-A1- 2008 160 149

## Description

### OBJECT OF THE INVENTION

The present invention relates to a method for reducing and/or eliminating a target agent from a fluid, said fluid being pumpable, and said fluid preferably being a liquid food flow rate. The invention is set out in the appended set of claims.

Preferably, the present method makes it possible to sterilise and/or pasteurise a food in a liquid state, to ensure the safety of the food during its processing, reducing and/or eliminating the target agent present in said liquid food flow rate based on the control (that is, recording, monitoring and verification) of a control measure, in addition to the modification of the parameters that define said sterilisation and/or pasteurisation process. The control measure of a heat treatment, particularly a pasteurisation and/or sterilisation process, is referred to as the set of specific combinations *"Treatment temperature - treatment time"* which is applied to the liquid food flow rate, characterised by a single parameter, referred to as the cumulative case fatality rate or F value or P value.

The present invention, therefore, relates to the regulation and control of the microbial load, enzymatic load, or of any other biochemical or chemical load, of the pumpable fluid through a heat treatment, preferably a pasteurisation and/or sterilisation process, carried out on the liquid food flow rate. That is, the present method carries out regulation and control of the values of the load level of the target agent present in said liquid food flow rate, thus ensuring that the target value (n_{threshold}) of the target agent, depending on the heat treatment applied, said target value (n_{threshold}) being the specific threshold number of log-decimal reductions in the initial load of the target agent, or safety target agent, present in the food.

### BACKGROUND OF THE INVENTION

In the state of the art, there is extensive knowledge about heat treatments in food flow rates, such as pasteurisation treatments, and systems that allow the application of said treatments, based on a holding time and temperature of the liquid food flow rate sufficient for said treatment to provide a case fatality rate of the target agent which meets the objective of heat treatment, particularly pasteurisation and/or sterilisation.

However, the methods known today carry out the heat treatment at issue based on preset and constant values of holding time and holding temperature, without knowing at each instant throughout the process the cumulative case fatality rate provided by said heat treatment, that is, the cumulative case fatality rate supplied by the system that applies said treatment at each instant, by means of the treatment temperature-time combination applied.

Due to oscillations in the holding temperature and flow rate to which the system for applying the heat treatment is subjected, it is not possible to know exactly and in real time the cumulative case fatality rate provided by said system against the target agent present in the liquid food flow rate for each applied treatment temperature-time combination recorded at each instant.

That is, the equipment that makes it possible to carry out heat treatments such as those indicated do not have automated systems that make it possible to reliably warn whether or not the combination of the holding temperature and/or flow rate values (related to the holding time) of said treatment provides a sufficient case fatality rate against the target agent that is intended to be eliminated or reduced, in order to exceed or not exceed the preset limit threshold value of said target agent, as the heat treatment is applied.

In this way, in the current equipment for applying heat treatments, such as pasteurisation and/or sterilisation, the efficacy of this treatment is unknown when the liquid food flow rate and the holding temperature vary and, therefore, it is not possible to regulate the parameters of the treatment in order to obtain the required efficacy.

Moreover, the lack of control of the efficacy of the heat treatment carried out on the liquid food flow rate implies that, although the treatment does not obtain the required values of the case fatality rate of the target agent, the liquid food flow rate leaves the heat treatment under certain inadequate conditions, without the possibility of modification and resulting in a waste of product by not meeting the predetermined objectives of safety or quality in food.

This implies, therefore, that currently known methods allow the application of a heat treatment to a fluid flow rate in order to try to reduce the volume of the microbial, enzymatic, biochemical or chemical load, of a target agent present or potentially present in the fluid, without the possibility of knowing the efficacy of said heat treatment against the target agent, based on the variation during the process of applying said treatment of the different temperature and flow combinations recorded at each instant. Therefore, it is not possible to know the real efficacy of a treatment of this type at each instant.

Document WO 2008/132046 A1 discloses a UHT treatment of liquid foods susceptible to thermal damage.

Document EP 1106083 A1 discloses a method of pasteurizing with monitoring and controlling of the number of uptaken pasteurization units and an apparatus for performing such method.

### DESCRIPTION OF THE INVENTION

The present invention makes it possible to solve the aforementioned problems by means of a process for the reduction and/or elimination of a target agent from a fluid by means of a system that allows the application of said process.

The invention therefore relates to a method according to claim 1, namely *a method for reducing and*/*or eliminating a target agent from a fluid by means of a system comprising:*
- *a tube extending between a first end and a second end, forming an internal volume V according to a path,*
   *wherein the first end is a fluid inlet and the second end is a fluid outlet, wherein the fluid enters the internal volume V of the tube through the inlet of the tube and runs along the path to the outlet of the tube, wherein said fluid is driven by a pump,*
- *heating means fluidically connected to the inlet of the tube,*
- *at least one fluid flow rate sensor, configured to measure the flow rate Q of the fluid driven by the pump,*
- *at least one temperature sensor, located at the first end of the tube and*/*or at the second end of the tube and*/*or at any intermediate point of the path between the first end and second end of the tube, the at least one temperature sensor being configured to measure the temperature T of the fluid driven by the pump,*
- *control means, comprising a controller and storage means, the method comprising inter alia the following steps:*
   *a) providing the storage means of the control means with the following predetermined values:*
      - *t_{validated}, holding time of the fluid in the internal volume V of the tube, from its inlet to its outlet,*
      - *T_{validated}, temperature of the fluid at the outlet of the tube,*
      - *F_{target}*, *value of the case fatality rate required to achieve the required reduction and*/*or elimination of the target agent,*
   *b) introducing and circulating by means of the pump, during the holding time t_{validated}, the fluid at the temperature T_{validated} in the interior volume V of the tube,*
   c) *recording, by means of the at least one temperature sensor and the at least one flow rate sensor, for each instant of time tᵢ* , vi = 0, 1, ... , m, *the real temperature value of the fluid, Tᵢ and the real flow rate value Qᵢ, of the fluid in the interior volume V of the tube, the real temperature value of the fluid Tᵢ being a constant value at any instant of time tᵢ,*
   *d) calculating for each instant of time tᵢ, using the control means and based on the records of the flow value Qᵢ of the fluid, the real holding time tₘᵢ* of *the fluid obtained in the interior volume V of the tube, wherein:* ${t}_{mi} = \frac{V}{{Q}_{i}}$
   *e) calculating for each instant of time tᵢ, using the control means, the value of the case fatality rate provided by the system for the reduction and*/*or elimination of the target agent, Fᵢ, according to the values recorded and calculated in steps c) and d), by means of:* ${F}_{i} = {\int }_{{t}_{0}}^{tmi} {10}^{\left(\frac{{T}_{i} - {T}_{ref}}{Z}\right)} dt ,$ *wherein Z is a characteristic parameter of the target agent, particularly a* kinetic property of reduction and/or elimination of the target agent, *and T_{ref} is the chosen reference temperature to express the case fatality rate provided, Fᵢ,*
   *f) verifying for each instant tᵢ, using the control means, if condition Fᵢ < F_{target} is met, and*
   *g) regulating, using the control means, if the condition of step f) is met, the temperature value of the fluid Tᵢ and*/*or the flow rate value of the fluid Qᵢ* in the interior volume *V* of the tube, *which verify the condition Fᵢ* ≥ *F_{target}*.

In this way, the application of the present process on the target agent to be reduced and/or eliminated, present or potentially present in a fluid, is carried out through a system defined by means of a tube. In a particular embodiment, the tube is a continuous element, preferably manufactured in a single piece. In a particular embodiment, the tube is a continuous element, obtained by means of attaching a plurality of elements together, forming a tube once attached. Preferably, the elements are straight or curved tubes attached to each other consecutively by attachment means, preferably welding or mechanical couplings.

In a particular embodiment, said target agent is any microbial, enzymatic or biochemical agent, particularly, any microorganism present in the fluid that requires population control. In this way, any microbial, enzymatic or biochemical agent, particularly, any pathogenic or quality-altering microorganism that is more heat-resistant to the flora present or potentially present in food and can develop or survive in it, is referred to as a target agent, safety target agent or quality target agent.

The tube defines a path for the fluid, comprised between a first end and a second end of the tube. Said path is defined based on multiple configurations, the preferred configuration being a series of long, straight and parallel pipes, attached to one another by 180 °C bends.

The fluid therefore travels through the internal volume V defined by said tube, according to the path defined by it, entering the internal volume V through the inlet present at the first end, and leaving the interior of the tube through the outlet present at the second end of the tube. A pump, and particularly the drive power of said pump, allows the fluid to run along the path defined in the interior of the tube, said fluid therefore being pumped along its entire path.

The tube comprises a continuous section and a length, which defines its internal volume. *V*. In a particular embodiment, the section of the tube is constant.

Additionally, the system also comprises at least one fluid flow rate sensor, configured to measure the flow rate Q of the fluid driven by the pump within the internal volume V of the tube. In a particular embodiment, the at least one flow rate sensor is a flow meter that allows the fluid flow rate circulating through the tube to be measured.

Additionally, the system also comprises heating means fluidically connected to the tube, so that the fluid is heated prior to being introduced into the tube through the inlet present at the first end of said tube. Thus, the system raises the temperature of the fluid prior to its entry into the interior of the tube, and holds said temperature for a specific time within said tube, which provides the process with an efficacy in the objective of reducing and/or eliminating the target agent. In a particular embodiment, the outlet of the food flow rate from the heating means is connected in series with the inlet of the tube.

In a particular embodiment, the temperature of the fluid is raised in the heating means through steam injection or by indirect contact with any other hot fluid, through a heat exchanger.

Moreover, the system in turn comprises at least one temperature sensor, configured to record the temperature *Tᵢ* of the fluid in an instant of time *tᵢ,* ∀*i* = 0, 1, ..., *m* along its path through the internal volume V of the tube. In this way, the present method provides a single record of the temperature *Tᵢ* of the fluid in an instant of time *tᵢ* for calculating the case fatality rate provided *Fᵢ*.

The at least one sensor that provides said record *Tᵢ* it is located at any intermediate point of the path of the fluid, located between the first and the second end of the tube, or at the inlet and/or outlet of the fluid in said tube, that is, at the first and/or second end of the tube, respectively. In a particular embodiment, the at least one temperature sensor is a probe, which takes a direct measurement of the temperature of the fluid at the point where it is located.

In a particular embodiment, for the calculation of *Fᵢ* carried out in step e) at each instant of time *tᵢ*, this method preferably uses the temperature record *Tᵢ* coming from the probe located at the outlet of the tube, that is, at the second end of the tube. Advantageously, this temperature record is the most conservative, since the outlet of the tube is the most unfavourable point due to energy losses into the environment, as the fluid circulates through the tube from the inlet to the outlet.

The real temperature value of the fluid *Tᵢ* recorded in step c) is a constant value at any instant of time *tᵢ* . That is, the recorded temperature of the fluid particle entering the tube or exiting the tube remains constant as the fluid moves through the tube, regardless of whether the temperature sensor is located at the beginning or end of the tube. Thus, the system assumes that the temperature measured at the outlet of the tube has remained constant along its path through the tube, that is, from the inlet of the fluid in the tube to the outlet of the fluid in the tube, where the temperature *Tᵢ* recorded at the outlet of the tube is the temperature of the fluid along its path through the tube.

In this way, when the system has a single probe located at the outlet of the tube, the record of the temperature *Tᵢ* at any instant of time *tᵢ* will coincide with the real temperature of the fluid at the outlet of the tube. However, when the system does not have a temperature probe located at the outlet of the tube, but rather at the inlet or at any intermediate point between the inlet and outlet of the tube, the record of the temperature *Tᵢ* is considered to be the record coming from the probe located at the first end of the tube or at an intermediate point, as if it had been made at the second end of the tube, therefore considering the temperature *Tᵢ* at any instant of time *tᵢ* to be constant as the fluid circulates in the interior of the tube, that is, from the inlet of the fluid in the tube to the outlet of the fluid in the tube.

Finally, the system also comprises control means, said control means in turn including a controller configured to execute the control and regulation of the process, as well as storage means, configured to store data recorded by the at least one temperature sensor, as well as data related to the fluid present in the internal volume of the tube.

In a particular embodiment, the control means are configured to control the temperature of the fluid in the internal volume of the tube, as well as the holding time of the fluid in the internal volume of said tube, that is, the time it takes the fluid to travel through the entire internal volume V of the tube.

In a particular embodiment, the tube defines an internal volume V which, together with the fluid flow rate *Q* circulating through said internal volume V, makes it possible to obtain the minimum time necessary *tₘ* for a fluid particle to meet the target reductions n_{threshold} running along the entire internal volume *V* of the tube, where the minimum time necessary *tₘ* is the time calculated based on the fluid flow rate measured at the outlet of the tube.

The mentioned system makes it possible to carry out the method according to the invention. Said process begins by incorporating a series of predetermined values into the storage means. These predetermined values are setpoint values, that is, working values of the system that have previously been determined in order to establish the treatment to be applied to the fluid by the system. Said previously calculated values are incorporated into the system as working setpoints to be followed by the system. The predetermined values to be provided to the system to begin its operation are the following:
- *t_{validated}*: the holding time of the fluid in the internal volume *V* of the tube, from the inlet to the outlet of the fluid inside said tube. That is, the time it takes the fluid to run along the entire path defined by the tube. Said holding time is the minimum time established for the heat treatment carried out on the fluid housed in the internal volume *V* of the tube to be effective. This holding time is introduced into the system indirectly by means of the flow rate, taking into account the volume of the tube.
- *T_{validated}*: the temperature of the fluid at the outlet of the internal volume *V* of the tube. That is, the outlet temperature of the fluid from inside the tube, after being subjected to the heating means, that is, after being subjected to the previous section of the system that allows the fluid to be heated up to the temperature required for the heat treatment carried out on the fluid to be effective.
- *F_{target}*: value of the target or required case fatality rate to eliminate and/or reduce the target agent.

Once the previous predetermined parameters have been defined, the fluid is introduced into the interior volume V of the tube, and its circulation is caused by the pump, during the predetermined holding time *t_{validated}* at the predetermined temperature *T_{validated}.*

Subsequently, the temperature of the fluid *Tᵢ* and the value of the flow rate volume *Qᵢ* for an instant of time *tᵢ ,* ∀*i* = 0,1, ..., *m* are recorded on the fluid introduced into the tube through the at least one temperature sensor and the at least one flow rate sensor, where the recording point and therefore the location point of the at least one temperature sensor and of the at least one flow rate sensor is any point located between the inlet of the fluid into the interior of the tube until its outlet from the interior of said tube at its second end.

That is, the process records in the storage means the value of the temperature *Tᵢ* and the flow rate *Qᵢ* for an instant of time *tᵢ* , ∀i = 0,1, ..., m, where *t*₀ is the instant of time in which the fluid enters the interior of the tube and *tₘᵢ* is the holding time, or instant in which the fluid exits from the interior of the tube.

In a particular embodiment, the value of the temperature *Tᵢ* recorded is *Tₘ* , that is, the temperature at the instant of time *tₘ* when the fluid exits from the interior of the tube.

In a particular embodiment, the process records in the storage means the value of various temperatures *Tᵢ* and various flow rate values *Qᵢ* for each instant of time *tᵢ* , ∀*i* = 0, 1, ..., *m*. Advantageously, this makes it possible to have a greater number of real data about the conditions of the treatment applied to the fluid, and therefore its efficacy. Through the records taken and the predetermined values, the present method makes it possible to calculate, using the control means and based on the temperature records *Tᵢ* and the flow rate value *Qᵢ* of the fluid, the holding time *tₘᵢ* of the fluid in the interior volume V of the tube, wherein: ${t}_{mi} = \frac{V}{{Q}_{i}}$

That is, at each instant of time *tᵢ* in which a record is made, the holding time is obtained *tₘᵢ* based on the flow rate *Qᵢ*, and the temperature *Tᵢ* of each particle of the fluid that leaves the second end of the tube in that instant.

According to the invention, step d) further comprises calculating the holding time *tₘᵢ* factored by a magnification factor FM considering the circulation regime of the fluid. Said magnification factor FM depends on the factored speed of the fluid along the tube, thus establishing a conservative criterion for determining the heat treatment to be applied.

Likewise, this method makes it possible to calculate, using the control means, the value of the case fatality rate provided *Fᵢ* against the target agent at each instant of time *tᵢ*, which is defined by means of: ${F}_{i} = {\int }_{{t}_{0}}^{{t}_{mi}} {10}^{\left(\frac{{T}_{i} - {T}_{ref}}{Z}\right)} dt ,$ wherein Z is one of the kinetic properties of reduction and/or elimination of the target agent and *T_{ref}* is the chosen reference temperature with respect to which the case fatality rate, *Fᵢ*, is to be expressed. That is, the reference temperature *T_{ref}* is the temperature used as a reference for the comparison of its heat treatment with the heat treatment carried out by the present process.

In this way, a target agent having kinetic parameters D_{TREF} and Z (where D_{TREF} and Z are specific parameters of the target agent) is considered.

Moreover, a reference treatment with a unitary value is considered and, with said unitary reference treatment preferably having a duration (holding time) of 1 minute, it is used to compare the current heat treatment.

To define said reference treatment, preferably of a unitary value, this being 1 minute, the kinetic parameters D_{TREF} and Z are considered (where D_{TREF} and Z are, in this case, specific values of this reference agent that has served to define the reference unitary treatment).

In a particular embodiment, step e) further comprises calculating the case fatality rate provided against the reference agent, *Fᵢ'*. ${F}_{i} ′ = {\int }_{{t}_{0}}^{{t}_{mi}} {10}^{\left(\frac{{T}_{i} - {T}_{ref}}{Z}\right)} dt ,$ wherein Z is a specific value of the reference agent used to define the reference treatment and *T_{ref}* is the chosen reference temperature with respect to which the case fatality rate, *Fᵢ'*, is to be expressed, which in this method coincides with the reference temperature chosen to define the case fatality rate *Fᵢ*. That is, *T_{ref}* is constant for the calculation of both *Fᵢ'* and *Fᵢ'*.

The system makes a distinction between the target agent and reference agent. In this way, the target agent indicates that agent, preferably a pathogenic or quality-altering microorganism, against which it is desired to validate the heat treatment carried out by the present process with respect to the safety or quality of the processed fluid, particularly processed food. On the other hand, the reference agent is that agent, fictitious or real, from which its kinetic values are taken, particularly its reference thermal destruction kinetic parameters, *D_{Tref}* and Z.

The case fatality rate F is also technically known by other names such as: Sterilising value, fatality at T_{ref}., F_{Tref}^{Zref} or P_{Tref}^{Zref}.

In a particular embodiment, the cumulative case fatality rates *Fᵢ* are used as indicators of the degree of pasteurisation/sterilisation supplied by the system to the fluid, particularly a food, through the heat treatment applied by the present process.

By way of example, the cumulative case fatality rate known in the industry as F₀ = F_{121.1°C}^{10.0°C} indicates the reference treatment applied to the sporulated form of a generic serotype of the bacterium *Clostridium botulinum.* The kinetic value Z for the sporulated form of this bacterium, which serves to define the cumulative case fatality index F₀, is 10°C. Considering a reference temperature T_{ref}. = 121.1 °C, the decimal reduction time D_{Tref}. is 0.25 minutes, which consequently corresponds to a reference treatment of: F_{121.1°C}^{10.0°C} =F₀ = 3 minutes at 121.1 °C to achieve 12 log₁₀ reductions of the reference agent.

In a particular embodiment, in step e) the target agent and the reference agent may coincide and, accordingly, the values of the case fatality rates will also coincide, that is *Fᵢ* = *F'ᵢ.*

Once the value of the case fatality rate provided against the target agent *Fᵢ* at the instant of time *t* has been calculated, it is compared with the value of the target case fatality rate that is required, predetermined and entered in the first step, *F_{target},* so that the present method makes it possible to evaluate if the treatment applied to the fluid is correct and effective at the instant of time *tᵢ*.

In a particular embodiment, the present process compares, for each instant of time *tᵢ* in which a fluid temperature record *Tᵢ* has been made, the calculated value of the case fatality rate provided against the target agent *Fᵢ* in the instant of time *tᵢ* with the value of the target case fatality rate that is required, predetermined and entered in the first step, *F_{target},* so that the present method makes it possible to evaluate if the treatment applied to the fluid is correct and effective at the instant of time *tᵢ*.

In this way, step f) of the present method verifies, using the control means, if *Fᵢ* < *F_{target},* that is, if the value of the case fatality rate provided by the system by means of the heat treatment applied at the instant of time *tᵢ* exceeds the value of the case fatality rate *F_{target}* for the control of the fatality supplied against the target agent.

If the condition *Fᵢ* < *F_{target}* is verified, then the treatment applied to the fluid at the instant of time *tᵢ* is not considered effective, as the value of the case fatality rate provided *Fᵢ* is lower than the value of the minimum case fatality rate *F_{target}* determined that is required, preferably previously validated and, therefore, required for adequate fluid treatment.

In this case, the present method carries out step g), whereby the necessary parameters, in this case the temperature value of the fluid *Tᵢ* and/or flow rate value *Qᵢ* of the fluid in the interior volume V of the tube, are regulated in order to verify the condition *Fᵢ* ≥ *F_{target}.*

Advantageously, the present method allows a control and regulation of the treatment applied to the fluid, checking the efficacy of the treatment and the scope of the target agent at each instant. That is, the present method optimises the process of reducing and/or eliminating a target agent in the fluid, regulating the operation of the system based on the joint regulation of the holding time and the holding temperature, as an indirect regulation of the value of the case fatality rate provided by the system *Fᵢ*.

In a particular embodiment, step g) of the present method is carried out by means of a PID algorithm of the controller of the control means.

That is, the PID algorithm allows the control of the system through the control of the established parameters. Said PID algorithm is integrated in the control means of the system, and allows the regulation of the temperature of the fluid *Tᵢ*, among other possible parameters such as flow rate *Qᵢ*.

In a particular embodiment, the PID algorithm receives, as part of the parameters to be controlled, the value of the target or required case fatality rate, *F_{target},* additionally calculating the value of the case fatality rate provided, *Fᵢ*, in the recorded instant(s) and comparing the values in order to establish if the treatment carried out on the fluid is adequate.

According to the invention, in which the value of the temperature of the fluid *Tᵢ* is constant during the treatment holding time of the fluid in the tube, step d) of the present method comprises calculating the holding time *tₘᵢ* of the fluid in the interior volume V of the tube, where: ${t}_{mi} = \frac{{V}_{M}}{{Q}_{i}}$

Said calculation of the holding time *tₘᵢ* is increased in this particular embodiment by a magnification factor FM considering the food circulation regime, thus establishing a conservative criterion for determining the heat treatment to be applied.

In the present particular embodiment, the fastest food particle is considered for each of the records of the flow rate value *Qᵢ* for each instant of time *tᵢ*.

In this way, in the present particular embodiment, the fluid regime is obtained from the mean circulation speed of the fluid in the tube and the calculation of the Reynolds number (Re), in turn obtained based on the viscosity and density data of the fluid flowing through the tube.

In the present particular embodiment, when Re<4000, it is considered that the fluid circulates in a laminar regime, while when Re≥ 4000, it is considered that the fluid circulates in a turbulent regime.

Additionally, based on the previously calculated Reynolds number (Re), the flow behaviour index (n) of the fluid (characteristic parameter of the fluid and therefore known), as well as the density and viscosity of said fluid, the magnification factor FM of the already calculated mean circulation speed is obtained. This magnification factor FM makes it possible to calculate the factored speed as follows:
- For 0<Re<4000 (laminar flow), the following is met:
   - Newtonian fluids (n=1); $FM = \frac{{v}_{max}}{{v}_{mean}} = 2$
   - Non-Newtonian fluids (n≠ 1); $FM = \frac{{v}_{max}}{{v}_{mean}} = \frac{3 n + 1}{n + 1}$
- For 4000≤Re≤35000 (turbulent flow), the following is met:
   - Newtonian fluids (n=1); $FM = \frac{{v}_{max}}{{v}_{mean}} = \frac{f \left(Re\right)}{{v}_{mean}}$; *where* $f \left(Re\right) = \frac{\frac{1}{\left(0.711-0.05975log{\left(Re\right)}^{2}\right)}}{1 - 0.09171 log {\left(Re\right)}^{2} + 0.00059 log {\left(Re\right)}^{4}}$
- For Re>35000 (turbulent flow), the following is met:
   - Newtonian fluids (n=1); $FM = \frac{{v}_{max}}{{v}_{mean}} = \frac{\frac{1}{0.0336 {log}_{10} {v}_{mean} + 0 , 662}}{{v}_{mean}}$

Likewise, the calculation ratio of step e), relating to the value of the case fatality rate provided, *Fᵢ*, is obtained as follows: ${F}_{i} = {10}^{\left(\frac{{T}_{i} - {T}_{ref}}{Z}\right)} {\int }_{{t}_{0}}^{{t}_{mi}} dt$

In a particular embodiment, the present method comprises a validation step a.1) for validating the values of step a). Said validation step advantageously makes it possible to establish the working conditions of the present process.

That is, established during the validation step are the following conditions or predetermined and necessary values of the following variables:
- *t_{validated}*: the holding time of the fluid.
- *T_{validated}*: the temperature of the fluid at the outlet of the tube.
- *F_{targer}*: the value of the required target case fatality rate.

In validation step a.1), these values are obtained through an inference process in a validation graph, wherein the validation of the control measure is carried out, said control measure being the cumulative case fatality rate, making it possible to deductively determine the predetermined values, or setpoint values, of temperature *T_{validated}* and flow rate volume or holding time *t_{validated}* which ensure that the acceptable threshold (n_{threshold}) of log₁₀ reductions of count n of the target safety agent (target agent) is equalled or exceeded, thus defining the required target case fatality rate *F_{target}.*

In a particular embodiment, the validation graph allows the modification and regulation of five parameters at will of the user, wherein each modification is shown in the validation graph, as is its influence over the other parameters and over the achievement of the target threshold of log₁₀ reductions of safety or quality count *n* to be achieved (n_{threshold}) in order to validate the process. The parameters that allow modification are:
- Holding temperature at the outlet of the tube.
- Holding time in the tube considering the fastest particle of the fluid, particularly of the food, (PMRA),
- Fluid flow rate,
- Initial target agent population in the food before heat treatment.
- Efficacy of the treatment against the target agent (n._{target}), wherein n_{target} are the decimal logarithmic reductions obtained against the target agent by the temperature-time combination chosen in the validation process.

In a particular embodiment, validation step a.1) of the treatment carried out by the present process, comprises graphically inferring the holding temperature and holding time values that make it possible to achieve that the cumulative case fatality rate provided by the system that carries out the present process on the fluid equals or exceeds the fatality value required to achieve that the efficacy of the process (n_{target}) reaches the value n_{threshold} established as an objective to guarantee the safety or quality of the fluid, particularly of the processed food.

In this way, the system receives to carry out step a) of the process, the holding temperature value *T_{validated}* and holding time value *t_{validated}* of the fluid already validated during this step a.1), in addition to the value of the required target case fatality rate *F_{target}* using these values in steps f) and g) of the process.

In a particular embodiment, the verification step f) can be performed graphically, using a graph similar to the validation graph, which also represents the values for each instant of time *tᵢ* of the case fatality rates provided at the reference temperature against the target agent and reference agent and the holding temperature and holding time records recorded at each instant of time *tᵢ.*

In a particular embodiment, the present method comprises the previous step of sterilising the tube of the system, as well as possible additional cooling means for the fluid that are connected after the tube, that is, fluidically connected to the outlet of the tube, reducing and/or eliminating a target agent present in said tube and/or in said additional cooling means, by means of a sterilisation fluid, preferably by means of steam or hot water.

Advantageously, this allows the sterilisation of the internal volume of the system and of the tube through which the fluid circulates during the treatment. Additionally, it also allows the sterilisation of additional cooling means which are fluidically connected to the outlet of the tube.

In a particular embodiment, the previous step of sterilising the tube comprises the objective of maintaining the system above a preset threshold temperature in a determined temperature probe for the time strictly necessary to reach a specific value of the preset cumulative case fatality rate F₀.

Thus, in the present particular embodiment, the previous sterilisation step is performed by calculating the cumulative case fatality rate F₀, using as a temperature probe any one belonging to the tube. This temperature probe is the one that provides the records *Tᵢ* for calculating the cumulative case fatality rate F_{0:} ${F}_{0} = {\int }_{{t}_{0}}^{{t}_{mi}} {10}^{\left(\frac{{T}_{i} - {T}_{ref}}{Z}\right)} dt ,$ wherein Z= 10 °C is a characteristic kinetic parameter of the microorganism *Clostridium botulinum and T_{ref}*= 121.1 °C, the chosen reference temperature to express the case fatality rate of the reference treatment.

In a particular embodiment, the temperature sensor can be located in cooling means connected after the tube, that is, fluidically connected to the outlet of the tube.

In this case, the system performs the verification of compliance with the objective of reaching the preset value F₀ to complete sterilisation of either the tube of the system, or the tube and the cooling means of the system, and calculates the time elapsed to reach the cumulative case fatality rate F₀ In a particular embodiment, the value of the cumulative case fatality rate F₀ reached during the sterilisation of the equipment is obtained by numerical integration according to the trapezoidal method.

In a particular embodiment, the previous step of sterilising the tube comprises a first step of incorporating a series of predetermined values into the storage means of the control means.

These predetermined values are setpoint values, that is, working values of the system that have previously been determined in order to establish the sterilisation treatment to be applied. Said previously calculated values are incorporated into the system as working setpoints to be followed by the system, where these values are:
- F_{validated sterilisation}: Value of the target sterilisation index, that is, the value of the target case fatality rate, obtained from the data of the target agent considered to previously sterilise the equipment. In this way, the minimum case fatality rate to be achieved, either in the tube and/or in the cooling means located behind the tube, is established.
- T_{start sterilisation}: temperature value in at least one given temperature sensor from which the cumulative sterilisation index is calculated. Said temperature sensor can be located, as previously indicated, either in the tube and/or in the cooling means located after the tube, where the point at which said sensor is located is the temperature recording point.

Once the above predetermined parameters have been defined, the sterilising fluid, particularly steam or hot water, is introduced into the interior volume V of the tube and circulated by means of the pump. It is necessary for said sterilising fluid to be at the predetermined temperature T_{start sterilisation}, so that the temperature of said sterilising fluid is controlled and regulated until reaching the indicated value of the temperature T_{start sterilisation}. Only from the instant in which the sterilising fluid has reached the mentioned value, is the value of the cumulative case fatality rate F₀ calculated. That is, it is not possible to start recording the temperatures of the sterilising fluid until the temperature of said fluid reaches the predetermined value in the previous step.

Subsequent, the temperature of the fluid *Tᵢ* for an instant of time *tᵢ* , ∀*i* = 0, 1, ..., *m* is recorded on the fluid introduced into the tube, through at least one temperature sensor. In particular, the recording point of said temperature of the fluid *Tᵢ* is the location point of the at least one temperature sensor by means of which said recording is made, the at least one temperature sensor being located either at any point between the inlet of the fluid into the tube to its outlet from the interior of said tube at its second end or in the cooling means located at the outlet of the tube, that is, in the cooling means after the tube. In particular, *t*₀ is the instant of time in which the temperature of the sterilising fluid is the temperature T_{start sterilisation}. Additionally, *tₘ* is the instant of time in which the cumulative case fatality rate equals the value of the target case fatality rate, that is, in which *F*₀ = *F*_{validated} sterilisation .

The sterilisation process then stores the temperature value *Tᵢ* in the storage means for each instant of time *tᵢ*. Based on previous records, sterilisation requires calculating the cumulative case fatality rate F₀ according to the ratio: ${F}_{0} = {\int }_{{t}_{0}}^{{t}_{mi}} {10}^{\left(\frac{{T}_{i} - {T}_{ref}}{Z}\right)} dt$

Wherein Z= 10 °C is a characteristic kinetic parameter of the microorganism *Clostridium botulinum and Tᵣₑₜ*= 121.1 °C, the chosen reference temperature to express the case fatality rate of the reference treatment.

Finally, the present sterilisation step determines if the value of the cumulative case fatality rate F₀ and the value of the validated sterilisation index F_{validated} sterilisation, meet the ratio F₀ >F_{validated} sterilisation, that is, the cumulative case fatality rate is higher than the value of the target sterilisation index. In the event that the present ration is met, it is considered that the sterilisation step provides a satisfactory result, so it is considered that the system has a sufficient level of sterility to start the step of applying heat treatment to the fluid, particularly, to the liquid food flow rate.

In a particular embodiment, the system further comprises a bypass valve, and wherein step g) of the method further comprises controlling the actuation of said bypass valve, particularly, acting on the opening and/or closing of the bypass valve.

In this way, the bypass valve allows diverting the fluid flow rate to prevent said fluid, particularly a food, from entering the packaging systems after the heat treatment provided in the tube, in the event that the fatality provided by the heat treatment has not been sufficient. This bypass valve can be located at the inlet or outlet of the tube.

In a second inventive aspect, the present invention defines a computer program configured to implement the method according to the first inventive aspect.

### DESCRIPTION OF THE FIGURES

To complete the description and for the purpose of aiding to better understand the features of the invention, a set of figures is attached to the present specification as an integral part thereof, in which the following is depicted in an illustrative and non-limiting manner:
Figure 1 shows a flowchart of a particular embodiment of a pasteurising/sterilising equipment for the application of a method for reducing and/or eliminating a target agent from a fluid, comprising a bypass valve located at the first end of the tube.
Figure 2 shows a flowchart of a particular embodiment of the pasteurising/sterilising equipment for the application of a method for reducing and/or eliminating a target agent from a fluid, comprising a bypass valve located at the second end of the tube.
Figure 3 shows a particular embodiment of a validation graph, with the process pending validation, that is, with the value of the case fatality rate provided by the current treatment lower than the value of the required target case fatality rate.
Figure 4 shows the validation graph of Figure 3 with the process validated, that is, with the value of the case fatality rate provided by the current treatment equal to the value of the required target case fatality rate.
Figure 5 shows a particular embodiment of a graphic verification and control process of the system of Figures 1 and 2.
Figure 6 shows a particular embodiment of a graphic verification and control process of the system of Figures 1 and 2.
Figure 7 shows a particular embodiment of a graphic verification and control process of the system of Figures 1 and 2.
Figure 8 shows a particular embodiment of the graph of the sterilisation process prior to the step of pasteurising/sterilising the food.
Figure 9 depicts the steps of this method applied to the liquid food as it passes through the tube, from its inlet to its outlet, in addition to the previous steps of sterilisation, validation and provision to the system of the data obtained in the previous step of validation.

### PREFERRED EMBODIMENT OF THE INVENTION

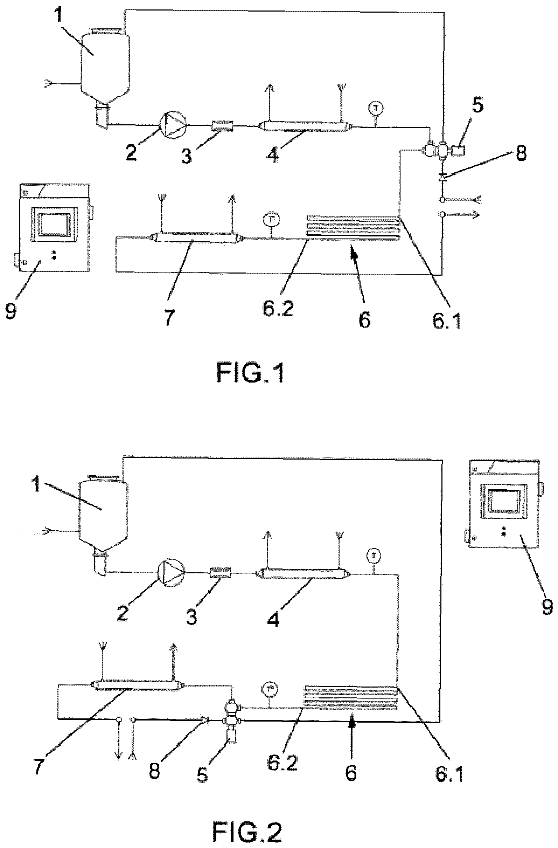

The present invention is schematically depicted in Figures 1 and 2. Said Figures 1 and 2 each shows a flowchart of the system used for the implementation of the steps of the method for reducing and/or eliminating a target agent from a fluid present in a liquid food, which is pumped through a tube by means of a fluid pump.

In this particular embodiment, the method also considers a reference treatment, said reference treatment being treatment F₀, widely known in the industry as an indicator of the cumulative case fatality of a microorganism.

Therefore, in a particular embodiment, the fatalities of the present method will be expressed against the reference treatment using its own parameters, T_{ref}=121.1 °C and Z=10 °C and against the target agent, or target microorganism, designated as *Bacillus stearothermophilus* using random parameters Dt = 1000 seconds and Z=7.3 °C.

Thus, both Figure 1 and Figure 2 show a system comprising a tank (1) containing the pumpable food to be treated by means of the corresponding heat treatment for the reduction of the target microorganism *Bacillus stearothermophilus.* The liquid food flow rate is pumped from the tank (1) by means of the pump (2) to a first heat exchanger (4), where its temperature will be increased up to a predetermined value by means of heat exchange with a heating fluid.

After the liquid food flow rate leaves the first heat exchanger (4), said flow rate is pumped until it enters the tube (6). A flow meter (3), located between the pump (2) and the heat exchanger (4), makes it possible to measure the flow rate Q of the pumpable food. Additionally, a temperature sensor measures the temperature T of pumpable food flow rate before it enters the tube (6), that is, before the fluid enters the tube (6) through its inlet (6.1).

Additionally, at the outlet of the tube (6), the pumpable food is introduced into a second heat exchanger (7), by means of which the temperature of said pumpable food is reduced through heat exchange with a cooling fluid. Previously, a temperature sensor measures the temperature T' of the pumpable food flow rate after it exits from the tube (6) through its outlet (6.2).

After the cooling obtained in the second heat exchanger (7), the pumpable food, already treated in the interior of the tube (6), is packaged in packaging equipment.

Additionally, this system comprises a bypass valve (5), located in the case of Figure 1 at the first end of the tube (6), while in Figure 2 it is located at the second end of the tube (6). Both systems also include a non-return valve (8), which prevents diverting the pumpable food after the bypass valve (5) through undesired paths.

Figures 1 and 2 also show control and storage means (9), which make it possible to carry out the different steps of the present method, particularly those calculations necessary for the different steps.

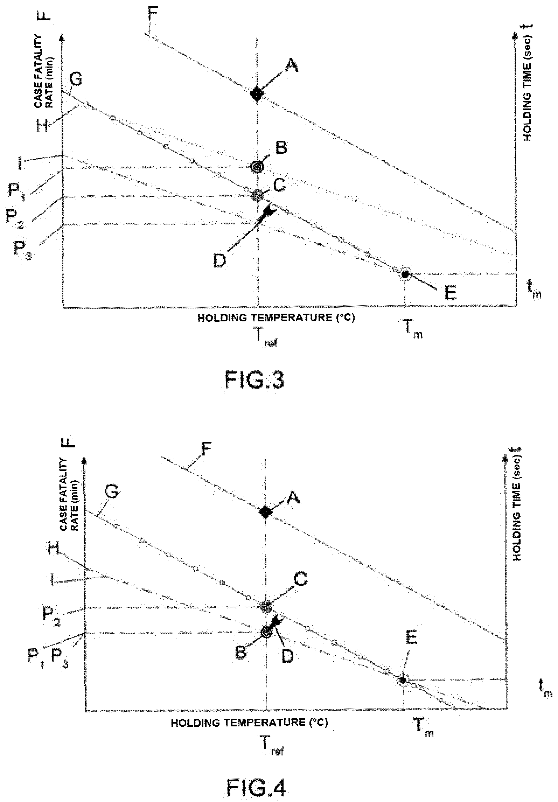

Figure 3 shows a validation graph, where the value of the case fatality rate provided by the current treatment is lower than the value of the required target case fatality rate, that is, *Fᵢ* < *F_{target}.* This indicates that the process, and therefore its predetermined values, have not been validated.

As this figure shows, point B is the minimum required F value or P value against the target agent at the reference temperature *T_{ref},* in order to meet the objective of safety and/or quality of the target agent from the present heat treatment, while point E represents the current pasteurisation/sterilisation treatment against the target agent and the reference agent, referenced with respect to holding temperature Tₘ and holding time tₘ.

In order for the validation criterion to be met, that is, for the value of the case fatality rate provided to be equal to the value of the target or required case fatality rate, that is, Fᵢ =F_{target}, it is necessary for said point B to coincide with point D, which represents the current treatment against the target agent, as depicted in Figure 4.

In combination with these points, it is also necessary to take into account point D, which represents the F value or P value of the treatment equivalent to the current treatment at the reference temperature *T_{ref},* as well as point C, which represents the F value or P value provided by the system at the reference temperature *T_{ref}* against the reference agent.

Additionally, point A represents the unitary reference treatment (equal to one minute), that is, the unitary F value or P value.

Finally, the points of intersection with the vertical axis, that is, with the value of the case fatality rate, represent the following:
- P₁: threshold fatality required against the target agent,
- P₂: threshold fatality against the reference agent, and
- P₃: fatality provided against the target agent.

These points are also present in Figures 4 to 7.

Figure 4 shows, for the same validation graph, that point B and point D coincide. In this case, the process, and therefore its predetermined values, have been validated, showing said coincidence.

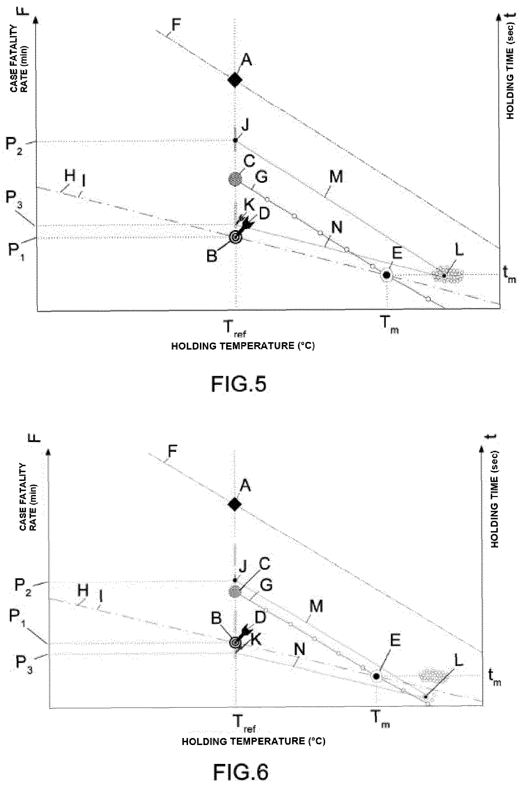

Figure 5 shows a particular embodiment of a graphic verification and control process of the system, where the values of the case fatality rates provided against the target agent (Fi) and against the reference agent (Fi') are represented.

It can also be observed in this particular embodiment that the value of the case fatality rate provided against the target agent (Fi) exceeds the value of the required target case fatality rate F_{target}, already obtained in the previous validation process, shown in Figures 3 and 4, and where it can also be seen that the value of the case fatality rate provided against the reference agent (Fi') is also higher than the value obtained in the previous validation step F_{target}.

Graphically, the points depicted in Figure 5 are the following:
- Point D: represents the F value or P value of the treatment equivalent to the current treatment at the reference temperature *T_{ref}* against the target agent,
- Point K: represents the current record of the F value or P value of the treatment equivalent to the current treatment at the reference temperature *T_{ref}* against the target agent,
- Point C: represents the F value or P value provided by the system at the reference temperature *T_{ref}* against the reference agent,
- Point J: represents the current record of the current F value or current P value of the system at the reference temperature *T_{ref}* against the reference agent,
- Point E: represents the pasteurisation/sterilisation treatment validated against the target agent and the reference agent, and
- Point L: represents the current pasteurisation/sterilisation treatment against the target agent and the reference agent.

Additionally, Figure 5 shows the following points:
- Point A: represents the F value or P value of the unitary reference treatment,
- Line I: represents the treatments equivalent to the current treatment against the target agent,
- Line G: represents the equivalent treatments against the current treatment against the reference agent,
- Line H: represents the threshold line delimiting valid treatments against the target agent,
- Line F: represents the line of reference treatments equivalent to the unitary one,
- Line N: represents the line of treatments equivalent to the current treatment against the target,
- Line M: represents the line of treatments equivalent to the current treatment against the reference agent.

In the case of Figure 6, for another particular embodiment of the graphic verification and control process, the values of the case fatality rates provided against the target agent (Fi) and against the reference agent (Fi') are observed, as is the fact that the value of the case fatality rate provided Fi is lower than the value of the required target case fatality rate F_{target} obtained in the previous validation process. The lines and points represented correspond to those mentioned in Figure 5.

However, it can also be seen that the value of the case fatality rate provided against the reference agent in this case is higher than the value obtained in the validation. In this particular embodiment, the safety or quality of the food is not guaranteed and the system would act on the controls to regulate the heating means and/or the flow rate until achieving Fi≥Fo.

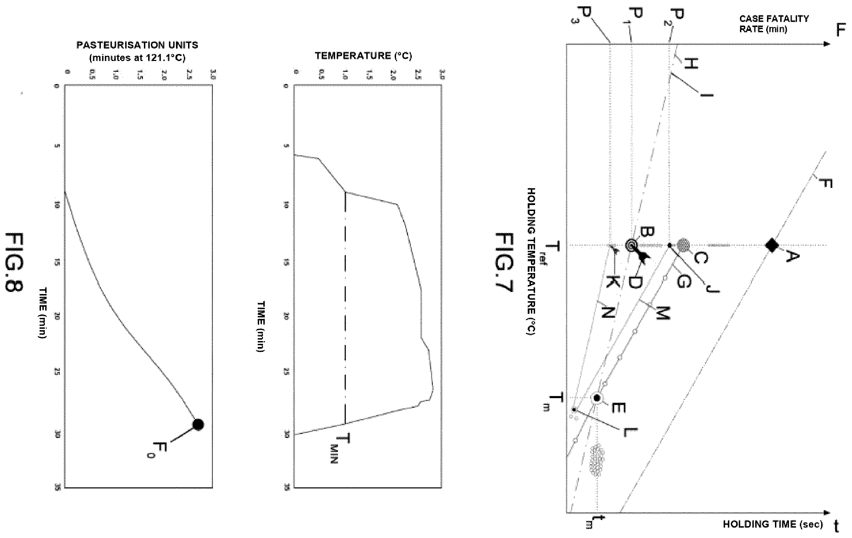

Lastly, Figure 7, relating to a particular embodiment of a graphic verification and control process, represents the values of the case fatality rates provided against the target agent (Fi) and against the reference agent (Fi'), while it is also observed that the value of the case fatality rate provided Fi is lower than the value of the required target case fatality rate F_{target} obtained in the previous validation process.

Additionally, this Figure 7 shows that the value of the case fatality rate provided against the reference agent in this case does not exceed the value obtained in the validation either. In this particular embodiment, the safety or quality of the food is not guaranteed and the system would act on the controls to regulate the heating means and/or the flow rate until achieving Fi≥Fo. The lines and points represented correspond to those mentioned in Figures 5 and 6.

Figure 8 shows an example of the graph of the sterilisation process prior to the step of pasteurising/sterilising the food.

Two graphs can be seen in this figure, the upper one relating to the evolution of the temperature of the fastest particle of the food over time, where the minimum temperature for which a sterilising effect of the system is obtained after applying the sterilisation step is also shown in broken line.

Additionally, the lower graph shows the evolution of the cumulative fatality provided by the system, in cumulative pasteurisation units provided from the start of the treatment until completing the total holding time. This evolution is shown by the continuous line present in the graph.

The same graph in turn shows a point that graphically determines the fatality of the pasteurisation treatment, or F₀.

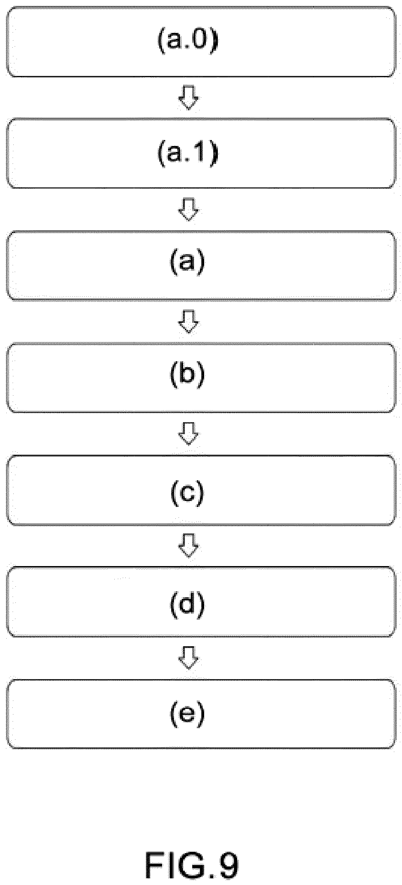

As can be observed in Figure 9, the flowchart shown indicates the steps of the method applied to a liquid food as it passes through the tube, from its inlet to its outlet.

In this case, prior to the entry of the liquid food into the tube, step a.0) of sterilising the tube is carried out, using steam or hot water. Said flow rate of steam or hot water is maintained at a temperature higher than the threshold value T_{start sterilisation} for a total time *tₘᵢ*, during which time the flow of steam or hot water is recirculating throughout the system, that is, in the interior volume of the tube to reduce its population of microorganisms once the preset or target value F₀ has been reached, reaching the desired level of sterilisation in the interior volume of said tube and in the cooling means after the tube, if any.

Next, the system performs a step a.1) of validating the variables required for the treatment, these being:
- *t_{validated}*: the holding time of the fluid = 28.3 seconds.
- *T_{validated}*: the temperature of the fluid at the outlet of the tube = 133.5 °C
- *F_{target}*: the value of the required target case fatality rate = 1413.66 seconds.

Subsequently, and once the values of these variables have been validated, step a) is carried out, wherein they are provided to the storage means of the control means, so that the treatment carried out on the liquid food inside the tube is based on these initial parameters, previously validated.

The system has temperature probes located at the inlet and/or outlet of the tube, which allow the recording of the temperature of the fluid *Tᵢ* for the instants of time *t* considered.

In the present embodiment, the system has a temperature probe at the outlet of the tube.

In this way, step b) of the method makes it possible, by means of the actuation of the probe at the outlet of the tube, to record in the storage means of the control means, the different values of the temperature of the fluid *Tᵢ* for the instants of time *t* considered. In addition, the method makes it possible, by means of the records coming from the flow rate probe, to calculate the holding time *tₘᵢ* of the fluid in the interior volume V of the tube, wherein: ${t}_{mi} = \frac{{V}_{M}}{{Q}_{i}}$

In the present example, the value of the volume necessary in the previous expression, based on the dimensions of the system, is Vₘ = 0.00785398 m³.

Said holding time *tₘᵢ* includes the calculation of the holding time factored by a magnification factor FM considering the circulation regime of the food, thus establishing a conservative criterion for determining the heat treatment to be applied. In the present particular embodiment, the fastest food particle is considered for each of the records of the flow rate value *Qᵢ* for each instant *tᵢ*.

The density and viscosity values used to calculate the FM in this exemplary embodiment are the following:
Density = 1.13 kg/l
Viscosity = 236 cP
Flow behaviour index (n) = 1
Reynolds number (Re) obtained: 16.93

Based on said temperature records of the fluid *Tᵢ* and of the holding time of the fastest particle *tₘᵢ,,* the control means of the system make it possible, in a step c) of the present method, to calculate the value of the case fatality rate of the target agent provided, *Fᵢ*, for each of the instants of time t considered.

In particular, the value of the case fatality rate provided, *Fᵢ*, is calculated based on the following ratio for the target agent, by the control means: ${F}_{i} = {\int }_{{t}_{0}}^{{t}_{mi}} {10}^{\left(\frac{{T}_{i} - {T}_{ref}}{Z}\right)} dt$

Where T_{ref} = 121.1 °C and Z = 7.3 °C.

On the other hand, the value of the case fatality rate provided compared to the reference agent, *F'ᵢ*, is calculated based on the following relationship by the control means: $F {′}_{i} = {\int }_{{t}_{0}}^{{tm}_{i}} {10}^{\left(\frac{{T}_{i} - {T}_{ref}}{Zref}\right)} dt$

Where T_{ref} = 121.1 °C and Z = 10 °C.

Once the value of the fatality rate provided, *Fᵢ,* at each instant of time t is known, the system allows the comparison of said values with the value of the required target case fatality rate, *F_{target},* previously determined in step a.1).

In this way, step d) of the present method carries out the comparison of values, checking if *Fᵢ* < *F_{target}* by means of the control methods.

In the event that the previous condition is met, the treatment carried out is not adequate to achieve the target value of reduction of the population of the target microorganism or target agent, so the control means carry out modifications in the configuration of the system to, thus, modify the parameters of the applied treatment, treatment temperature and/or flow rate or holding time and increase its efficacy on liquid food.

In this way, when step d) of the method is verified, that is, when the condition *Fᵢ* < *F_{target}* is verified, the control means could act on the bypass valve of the system, so that the inlet flow liquid food flow rate in the interior of the tube or at its outlet is diverted and returned to the previous storage tank from where the pump that circulates the fluid food is fed.

Thus, with these modifications of the parameters of the temperature and flow rate system, there are obtained additional records of the temperature of the fluid *Tᵢ* and holding time *tₘᵢ* for the instants of time t considered, and therefore modified values of the value of the case fatality rate provided *Fᵢ*, until the condition is verified *Fᵢ* ≥ *F_{target}*, which indicates that the applied treatment has the required efficacy.

In this particular embodiment, a series of two records are considered to evaluate the value of the case fatality rate in various situations:

| **Flow rate Qᵢ (I/h)** | **Temperature Tᵢ(°C)** | **Fastest particle residence time (s)** | **Fatality required against target agent F_{target}(s)** | **Fatality supplied against target agent (s)** | **Fatality supplied against the reference agent (s)** | **Number of log-decimal reductions of target agent n_{target agent}** |
|---|---|---|---|---|---|---|
| 355 | 137 | 39.823005468 | 1413.660307597 | 6000.897727348 | 1549.294693642 | 8.4898723 |
| 420 | 132.2 | 33.659921288 | 1413.660307597 | 1115.991896979 | 433.623785099 | 1.5788685 |

## Claims

1. A method for reducing and/or eliminating a target agent from a fluid by means of a system comprising:
- a tube (6) extending between a first end (6.1) and a second end (6.2), forming an internal volume V according to a path,
wherein the first end (6.1) is a fluid inlet and the second end (6.2) is a fluid outlet,
wherein the fluid enters the internal volume V of the tube through the inlet (6.1) of the tube (6) and runs along the path to the outlet (6.2) of the tube (6), wherein said fluid is driven by a pump (2),
- heating means (4) fluidically connected to the inlet (6.1) of the tube (6),
- at least one fluid flow rate sensor (3), configured to measure the flow rate *Q* of the fluid driven by the pump (2),
- at least one temperature sensor, located at the first end (6.1) of the tube (6) and/or at the second end (6.2) of the tube (6) and/or at any intermediate point of the path between the first end (6.1) and second end (6.2) of the tube (6), the at least one temperature sensor being configured to measure the temperature *T* of the fluid driven by the pump (2),
- control means (9), comprising a controller and storage means,
the method comprising the following steps:
a) providing the storage means of the control means with the following predetermined values:
• *t_{validated},* holding time of the fluid in the internal volume *V* of the tube, from its inlet to its outlet,
• *T_{validated},* temperature of the fluid at the outlet of the tube,
• *F_{target},* value of the case fatality rate required for the reduction and/or elimination of the target agent
b) introducing and circulating by means of the pump, during the holding time *t_{validated},* the fluid at the temperature *T_{validated}* in the interior volume *V* of the tube,
c) recording, by means of the at least one temperature sensor and the at least one flow rate sensor, for each instant *tᵢ*, ∀*i* = 0,1, ..., *m*, the real temperature value of the fluid *Tᵢ*, and the real flow rate value *Qᵢ*, of the fluid in the interior volume *V* of the tube, the real temperature value of the fluid *Tᵢ* being a constant value at any instant of time *tᵢ*,
d) calculating for each instant of time *tᵢ*, using the control means and based on the records of the real temperature *Tᵢ* and the real flow value *Qᵢ* of the fluid recorded in step c), the holding time *tₘᵢ* of the fluid in the interior volume V of the tube, wherein: ${t}_{mi} = \frac{V}{{Q}_{i}}$ wherein step d) further comprises calculating the holding time (*tₘᵢ*) factored by a magnification factor FM based on the circulation regime of the fluid, and wherein said magnification factor FM is calculated as described in page 12, line 13 to page 13, line 9 ,
e) calculating for each instant *tᵢ*, using the control means (9), the value of the case fatality rate provided, *Fᵢ*, according to the values recorded and calculated in steps c) and d), and wherein: ${F}_{i} = {\int }_{{t}_{0}}^{{t}_{mi}} {10}^{\left(\frac{{T}_{i} - {T}_{ref}}{Z}\right)} dt ,$ wherein Z is a kinetic property of the reduction and/or elimination of the target agent and *T_{ref}* the chosen reference temperature
f) verifying, for each instant *tᵢ*, using the control means (9), if the condition *Fᵢ < F_{target}* is met, and
g) regulating, using the control means (9), if the condition of step f) is met, the real temperature value of the fluid *Tᵢ* and/or the real flow rate value *Qᵢ* of the fluid in the interior volume *V* of the tube, which verifies the condition *Fᵢ* ≥ *F_{target}.*

2. Method for reducing and/or eliminating a target agent from a fluid according to claim 1, wherein step e) further comprises calculating the value of the case fatality rate provided against the reference agent, *Fᵢ'*, according to the values recorded and calculated in steps c) and d), and wherein: ${F}_{i} ′ = {\int }_{{t}_{0}}^{{t}_{mi}} {10}^{\left(\frac{{T}_{i} - {T}_{ref}}{Z}\right)} dt$ wherein Z is a specific value of the reference agent used to define the reference treatment and *T_{ref}* is the chosen reference temperature with respect to which the case fatality rate, Fi' is to be expressed.

3. Method for reducing and/or eliminating a target agent from a fluid according to any of the preceding claims, wherein step g) is carried out by means of a PID algorithm of the controller of the control means.

4. Method for reducing and/or eliminating a target agent from a fluid according to any of the preceding claims, comprising a step a.1) of validating the values of step a).

5. Method for reducing and/or eliminating a target agent from a fluid according to claim 4, wherein step a.1) is carried out based on a predetermined validation graph.

6. Method for reducing and/or eliminating a target agent from a fluid according to any of the preceding claims, wherein the verification step f) is carried out by means of a predetermined verification graph.

7. Method for reducing and/or eliminating a target agent from a fluid according to any of the preceding claims, further comprising the previous step of sterilising the tube of the system, reducing and/or eliminating a target agent present in said tube by means of a sterilising fluid, preferably by means of steam or hot water.

8. Method for reducing and/or eliminating a target agent from a fluid according to claim 7, wherein the previous step of sterilising the tube comprises the steps of:
i. providing the storage means of the control means with the following predetermined values:
- F_{validatedsterilisation}: Value of the target sterilisation index,
- Tₛₜₐᵣₜₛₜₑᵣᵢₗᵢₛₐₜᵢₒₙ: temperature value in at least one given sensor from which the cumulative sterilisation index is calculated,
ii. introducing the sterilising agent, preferably steam or hot water, into the interior volume *V* of the tube, and causing it to circulate by means of the pump, until reaching the temperature Tₛₜₐᵣₜ sterilisation,
iii. recording, by means of the at least one temperature sensor, the real temperature *Tᵢ* for an instant of time *tᵢ*,
iv. storing, in the storage means, the value of the real temperature *Tᵢ* recorded in iii) for each instant of time *tᵢ*
v. calculating the cumulative case fatality rate F₀ according to the ratio: ${F}_{0} = {\int }_{{t}_{0}}^{{t}_{mi}} {10}^{\left(\frac{{T}_{i} - {T}_{ref}}{Zref}\right)} dt$ wherein *Z_{ref}* is a reduction and/or elimination kinetic property of the reference agent and *T_{ref}* the chosen reference temperature with respect to which the case fatality rate F₀ is to be expressed,
vi. determining if the value of the cumulative case fatality rate F₀ and the value of the validated sterilisation index F_{validatedsterilisation}, meet the ratio F₀>F_{validatedsterilisation}, that is, the cumulative case fatality rate is higher than the value of the target sterilisation index established in step i).

9. Method for reducing and/or eliminating a target agent from a fluid according to any of the preceding claims, wherein the system further comprises a bypass valve, and wherein step g) of the method further comprises acting on the opening and/or closing of the bypass valve.

10. A computer program configured to implement the method according to any of claims 1 - 9 when it is run on the controller of the control means (9) of the system as defined in any of claims 1 - 9.

## Patentansprüche

1. Verfahren zum Reduzieren und/oder Eliminieren eines Zielmittels aus einem Fluid mittels eines Systems, umfassend:
- einen Schlauch (6), der sich zwischen einem ersten Ende (6.1) und einem zweiten Ende (6.2) erstreckt und ein Innenvolumen *V* gemäß einem Pfad bildet,
wobei das erste Ende (6.1) ein Fluideinlass ist und das zweite Ende (6.2) ein Fluidauslass ist,
wobei das Fluid in das Innenvolumen *V* des Schlauchs durch den Einlass (6.1) des Schlauchs (6) gelangt und entlang des Pfades zum Auslass (6.2) des Schlauchs (6) verläuft, wobei das Fluid von einer Pumpe (2) angetrieben wird,
- ein Heizmittel (4), das fluidisch mit dem Einlass (6.1) das Schlauchs (6) verbunden ist,
- mindestens einen Fluidströmungsmengensensor (3), der dazu konfiguriert ist, die Strömungsmenge *Q* des Fluids, das von der Pumpe (2) angetrieben wird, zu messen,
- mindestens einen Temperatursensor, der sich an dem ersten Ende (6.1) des Schlauchs (6) und/oder an dem zweiten Ende (6.2) des Schlauchs (6) und/oder an einem beliebigen Zwischenpunkt des Pfades zwischen dem ersten Ende (6.1) und dem zweiten Ende (6.2) des Schlauchs (6) befindet, wobei der mindestens eine Temperatursensor dazu konfiguriert ist, die Temperatur *T* des Fluids, das von der Pumpe (2) angetrieben wird, zu messen,
- ein Steuermittel (9), das eine Steuerung und ein Speichermittel umfasst,
wobei das Verfahren die folgenden Schritte umfasst:
a) Versehen des Speichermittels des Steuermittels mit den folgenden vorbestimmten Werten:
• *t_{validated}*, Haltezeit des Fluids in dem Innenvolumen *V* des Schlauchs von seinem Einlass zu seinem Auslass,
• *T_{validated}*, Temperatur des Fluids an dem Auslass des Schlauchs,
• *F_{target}*, Wert der Letalität, der zur Reduzierung und/oder Eliminierung des Zielmittels erforderlich ist,
b) Einbringen und Zirkulierenlassen, mittels der Pumpe, während der Haltezeit *T_{validated}*, des Fluids bei der Temperatur *T_{validated}* in dem Innenvolumen *V* des Schlauchs,
c) Protokollieren, mittels des mindestens einen Temperatursensors und des mindestens einen Strömungsmengensensors, für jeden Zeitpunkt *t*_{*i*,} ∀*i* = 0,1, ... , *m*, des realen Temperaturwerts des Fluids *Tᵢ* und des realen Strömungsmengenwerts *Qi* des Fluids in dem Innenvolumen *V* des Schlauchs, wobei der reale Temperaturwert des Fluids *Tᵢ* ein konstanter Wert zu einem beliebigen Zeitpunkt *tᵢ* ist,
d) Berechnen, für jeden Zeitpunkt *tᵢ*, unter Verwendung des Steuermittels und auf Grundlage der Protokolle der in Schritt c) berechneten realen Temperatur *Tᵢ* und des realen Strömungswerts *Qᵢ* des Fluids, der Haltezeit *tₘᵢ* des Fluids in dem Innenvolumen *V* des Schlauchs, wobei: ${t}_{mi} = \frac{V}{{Q}_{i}}$ wobei Schritt d) ferner Berechnen der Haltezeit (*tₘᵢ*) umfasst, faktorisiert durch einen Vergrößerungsfaktor FM auf Grundlage des Zirkulationsschemas des Fluids, und wobei der Vergrößerungsfaktor FM wie auf Seite 12, Zeile 13 bis Seite 13, Zeile 9 beschrieben berechnet wird,
e) Berechnen, für jeden Zeitpunkt *tᵢ* unter Verwendung des Steuermittels (9), des Werts der bereitgestellten Letalität, *Fᵢ*, gemäß den in Schritt c) und d) protokollierten und berechneten Werten, und wobei: ${F}_{i} = {\int }_{{t}_{0}}^{{t}_{mi}} {10}^{\left(\frac{{T}_{i} - {T}_{ref}}{Z}\right)} dt ,$ wobei Z eine kinetische Eigenschaft der Reduzierung und/oder Eliminierung des Zielmittels und *T_{ref}* die ausgewählte Referenztemperatur ist,
f) Verifizieren, für jeden Zeitpunkt *tᵢ*, unter Verwendung des Steuermittels (9), ob die Bedingung *Fᵢ* < *F_{target}* erfüllt ist, und
g) Regulieren, unter Verwendung des Steuermittels (9), wenn die Bedingung von Schritt f) erfüllt ist, des realen Temperaturwerts des Fluids *Tᵢ* und/oder des realen Strömungsmengenwerts *Qᵢ* des Fluids in dem Innenvolumen *V* des Schlauchs, die die folgende Bedingung verifizieren: ${F}_{i} \geq {F}_{target} .$

2. Verfahren zum Reduzieren und/oder Eliminieren eines Zielmittels aus einem Fluid nach Anspruch 1, wobei Schritt e) ferner Berechnen des Werts der bereitgestellten Letalität anhand des Referenzmittels, *Fᵢ*', gemäß den in Schritt c) und d) protokollierten und berechneten Werten umfasst, und wobei: ${F}_{i} ′ = {\int }_{{t}_{0}}^{{t}_{mi}} {10}^{\left(\frac{{T}_{i} - {T}_{ref}}{Z}\right)} dt$ wobei Z ein spezifischer Wert des Referenzmittels ist, der verwendet wird, um die Referenzbehandlung zu definieren, und *T_{ref}* die ausgewählte Referenztemperatur in Bezug darauf ist, wie die Letalität Fi' auszudrücken ist.

3. Verfahren zum Reduzieren und/oder Eliminieren eines Zielmittels aus einem Fluid nach einem der vorhergehenden Ansprüche, wobei Schritt g) mittels eines PID-Algorithmus der Steuerung des Steuermittels ausgeführt wird.

4. Verfahren zum Reduzieren und/oder Eliminieren eines Zielmittels aus einem Fluid nach einem der vorhergehenden Ansprüche, umfassend einen Schritt a.1) zum Validieren der Werte aus Schritt a).

5. Verfahren zum Reduzieren und/oder Eliminieren eines Zielmittels aus einem Fluid nach Anspruch 4, wobei Schritt a.1) auf Grundlage eines vorbestimmten Validierungsgraphen ausgeführt wird.

6. Verfahren zum Reduzieren und/oder Eliminieren eines Zielmittels aus einem Fluid nach einem der vorhergehenden Ansprüche, wobei der Verifizierungsschritt f) mittels eines vorbestimmten Verifizierungsgraphen ausgeführt wird.

7. Verfahren zum Reduzieren und/oder Eliminieren eines Zielmittels aus einem Fluid nach einem der vorhergehenden Ansprüche, ferner umfassend den vorherigen Schritt des Sterilisierens des Schlauchs des Systems, des Reduzierens und/oder Eliminierens eines Zielmittels, das in dem Schlauch vorhanden ist, mittels eines Sterilisierungsfluids, vorzugsweise mittels Dampf oder heißen Wassers.

8. Verfahren zum Reduzieren und/oder Eliminieren eines Zielmittels aus einem Fluid nach Anspruch 7, wobei der vorherige Schritt des Sterilisierens des Schlauchs die folgenden Schritte umfasst:
i. Versehen des Speichermittels des Steuermittels mit den folgenden vorbestimmten Werten:
- F_{validatedsterilisation}: Wert des Zielsterilisierungsindex,
- Tₛₜₐᵣₜₛₜₑᵣᵢₗᵢₛₐₜᵢₒₙ: Temperaturwert in mindestens einem bestimmten Sensor, anhand dessen der kumulative Sterilisierungsindex berechnet wird,
ii. Einbringen des Sterilisierungsmittels, vorzugsweise Dampf oder heißes Wasser, in das Innenvolumen *V* des Schlauchs und Bewirken, dass es mittels der Pumpe zirkuliert, bis die Temperatur T_{start sterilisation} erreicht ist,
iii. Protokollieren, mittels des mindestens einen Temperatursensors, der realen Temperatur *Tᵢ* für einen Zeitpunkt *tᵢ*,
iv. Speichern, in dem Speichermittel, des Werts der realen Temperatur *Tᵢ*, der in iii) für jeden Zeitpunkt *tᵢ* protokolliert wurde,
v. Berechnen der kumulativen Letalität *F₀* gemäß dem folgenden Verhältnis: ${F}_{0} = {\int }_{{t}_{0}}^{{t}_{mi}} {10}^{\left(\frac{{T}_{i} - {T}_{ref}}{Zref}\right)} dt$ wobei *Z_{ref}* eine kinetische Eigenschaft des Referenzmittels zur Reduzierung und/oder Eliminierung und *T_{ref}* die ausgewählte Referenztemperatur in Bezug darauf ist, wie die Letalität *F₀* auszudrücken ist,
vi. Bestimmen, ob der Wert der kumulativen Letalität F₀ und der Wert des validierten Sterilisierungsindex F_{validatedsterilisation} das Verhältnis F₀>F_{validatedsterilisation} erfüllen, das heißt, die kumulative Letalität höher als der Wert des Zielsterilisierungsindex ist, der in Schritt i) erzeugt wurde.

9. Verfahren zum Reduzieren und/oder Eliminieren eines Zielmittels aus einem Fluid nach einem der vorhergehenden Ansprüche, wobei das System ferner ein Umgehungsventil umfasst und wobei Schritt g) des Verfahrens ferner Wirken auf das Öffnen und/oder Schließen des Umgehungsventils umfasst.

10. Computerprogramm, das dazu konfiguriert ist, das Verfahren nach einem der Ansprüche 1-9 zu implementieren, wenn es auf der Steuerung des Steuermittels (9) des Systems nach einem der Ansprüche 1-9 ausgeführt wird.

## Revendications

1. Procédé de réduction et/ou d'élimination d'un agent cible d'un fluide au moyen d'un système comprenant :
- un tube (6) s'étendant entre une première extrémité (6.1) et une seconde extrémité (6.2), et définissant un volume interne *V* le long d'un trajet,
dans lequel la première extrémité (6.1) est une entrée de fluide et la seconde extrémité (6.2) est une sortie de fluide,
dans lequel le fluide pénètre dans le volume interne V du tube par l'entrée (6.1) du tube (6) et s'écoule le long du trajet jusqu'à la sortie (6.2) du tube (6), ledit fluide étant entraîné par une pompe (2),
- des moyens de chauffage (4) reliés fluidiquement à l'entrée (6.1) du tube (6),
- au moins un capteur de débit de fluide (3), configuré pour mesurer le débit Q du fluide entraîné par la pompe (2),
- au moins un capteur de température, situé au niveau de la première extrémité (6.1) du tube (6) et/ou au niveau de la seconde extrémité (6.2) du tube (6) et/ou en un point intermédiaire quelconque du trajet entre la première extrémité (6.1) et la seconde extrémité (6.2) du tube (6), l'au moins un capteur de température étant configuré pour mesurer la température *T* du fluide entraîné par la pompe (2),
- des moyens de commande (9), comprenant un dispositif de commande et des moyens de stockage,
le procédé comprenant les étapes suivantes :
a) la transmission, aux moyens de stockage des moyens de commande, des valeurs prédéterminées suivantes :
• *t_{validé}*, temps de maintien du fluide dans le volume interne V du tube, de son entrée à sa sortie,
• *T_{validée}*, température du fluide à la sortie du tube,
• *F*_{*cible*,} valeur du taux de létalité requis pour la réduction et/ou l'élimination de l'agent cible,
b) l'introduction et la mise en circulation au moyen de la pompe, pendant le temps de maintien *t_{validé}*, du fluide à la température *T_{validée}* dans le volume interne *V* du tube,
c) l'enregistrement, au moyen de l'au moins un capteur de température et de l'au moins un capteur de débit, pour chaque instant *t*_{*i*,}∀*i* = 0,1, ... , *m*, de la valeur de température réelle *Tᵢ* du fluide, et de la valeur de débit réel *Q_{i,}* du fluide dans le volume interne *V* du tube, la valeur de température réelle *Tᵢ* du fluide étant une valeur constante à tout instant de temps *t_{i,}*,
d) le calcul, pour chaque instant de temps *t_{i,}*, à l'aide des moyens de commande et sur la base des enregistrements de la température réelle *Tᵢ* et de la valeur de débit réel *Qᵢ* du fluide enregistrées à l'étape c), du temps de maintien *tₘᵢ* du fluide dans le volume interne *V* du tube, dans lequel : ${t}_{mi} = \frac{V}{{Q}_{i}}$ dans lequel l'étape d) comprend en outre le calcul du temps de maintien (*tₘᵢ*) multiplié par un facteur d'amplification FM fondé sur le régime de circulation du fluide, et dans lequel ledit facteur d'amplification FM est calculé tel que décrit dans la page 12, ligne 13 à la page 13, ligne 9,
e) le calcul, pour chaque instant *t*_{*i*,} à l'aide des moyens de commande (9), de la valeur du taux de létalité obtenue, *F*_{*i*,} en fonction des valeurs enregistrées et calculées aux étapes c) et d), et dans lequel : ${F}_{i} = {\int }_{{t}_{0}}^{{t}_{mi}} {10}^{\left(\frac{{T}_{i} - {T}_{ref}}{Z}\right)} dt ,$ dans lequel Z est une propriété cinétique de la réduction et/ou de l'élimination de l'agent cible et*T_{ref}* est la température de référence choisie,
f) la vérification, pour chaque instant *t*_{*i*,}, à l'aide des moyens de commande (9), du fait que la condition *Fᵢ* < *F_{cible}* est satisfaite, et
g) la régulation, à l'aide des moyens de commande (9), si la condition de l'étape f) est satisfaite, de la valeur de température réelle *Tᵢ* du fluide et/ou de la valeur de débit réel *Qᵢ* du fluide dans le volume interne *V* du tube, de manière à satisfaire la condition ${F}_{i} \geq {F}_{cible} .$

2. Procédé de réduction et/ou d'élimination d'un agent cible d'un fluide selon la revendication 1, dans lequel l'étape e) comprend en outre le calcul de la valeur du taux de létalité obtenue pour l'agent de référence, *Fᵢ*', en fonction des valeurs enregistrées et calculées aux étapes c) et d), dans lequel : ${F}_{i} ′ = {\int }_{{t}_{0}}^{{t}_{mi}} {10}^{\left(\frac{{T}_{i} - {T}_{ref}}{Z}\right)} dt$ dans lequel Z est une valeur spécifique de l'agent de référence utilisée pour définir le traitement de référence et*T_{ref}* est la température de référence choisie par rapport à laquelle le taux de létalité, *Fi'*, doit être exprimé.

3. Procédé de réduction et/ou d'élimination d'un agent cible d'un fluide selon l'une quelconque des revendications précédentes, dans lequel l'étape g) est mise en œuvre au moyen d'un algorithme PID du dispositif de commande des moyens de commande.

4. Procédé de réduction et/ou d'élimination d'un agent cible d'un fluide selon l'une quelconque des revendications précédentes, comprenant une étape a.1) de validation des valeurs de l'étape a).

5. Procédé de réduction et/ou d'élimination d'un agent cible d'un fluide selon la revendication 4, dans lequel l'étape a.1) est mise en œuvre sur la base d'un graphique de validation prédéterminé.

6. Procédé de réduction et/ou d'élimination d'un agent cible d'un fluide selon l'une quelconque des revendications précédentes, dans lequel l'étape de vérification f) est mise en œuvre au moyen d'un graphique de vérification prédéterminé.

7. Procédé de réduction et/ou d'élimination d'un agent cible d'un fluide selon l'une quelconque des revendications précédentes, comprenant en outre l'étape préalable de stérilisation du tube du système, réduisant et/ou éliminant un agent cible présent dans ledit tube au moyen d'un fluide de stérilisation, de préférence au moyen de vapeur ou d'eau chaude.

8. Procédé de réduction et/ou d'élimination d'un agent cible d'un fluide selon la revendication 7, dans lequel l'étape préalable de stérilisation du tube comprend les étapes suivantes :
i. la transmission, aux moyens de stockage des moyens de commande, des valeurs prédéterminées suivantes :
- F_{validéstérilisation} : valeur de l'indice de stérilisation cible,
- T_{débutstérilisation} : valeur de température dans au moins un capteur donné à partir duquel l'indice de stérilisation cumulatif est calculé,
ii. l'introduction de l'agent de stérilisation, de préférence de la vapeur ou de l'eau chaude, dans le volume interne *V* du tube, et sa mise en circulation au moyen de la pompe, jusqu'à ce que la température T_{début stérilisation} soit atteinte,
iii. l'enregistrement, au moyen de l'au moins un capteur de température, de la température réelle *Tᵢ* pour un instant de temps *t_{i,}*,
iv. le stockage, dans les moyens de stockage, de la valeur de la température réelle *Tᵢ* enregistrée en iii) pour chaque instant de temps *t*_{*i*,},
v. le calcul du taux de létalité cumulatif F₀ selon la relation : ${F}_{0} = {\int }_{{t}_{0}}^{{t}_{mi}} {10}^{\left(\frac{{T}_{i} - {T}_{ref}}{Zref}\right)} dt$ dans lequel *Z_{ref}* est une propriété cinétique de réduction et/ou d'élimination de l'agent de référence et *T_{ref}* la température de référence choisie par rapport à laquelle le taux de létalité F₀ doit être exprimé,
vi. la détermination du fait que la valeur du taux de létalité cumulatif F₀ et la valeur de l'indice de stérilisation validé F_{validéstérilisation} satisfont la relation F₀ > F_{validéstérilisation}, c'est-à-dire que le taux de létalité cumulatif est supérieur à la valeur de l'indice de stérilisation cible établie à l'étape i).

9. Procédé de réduction et/ou d'élimination d'un agent cible d'un fluide selon l'une quelconque des revendications précédentes, dans lequel le système comprend en outre une vanne de dérivation, et dans lequel l'étape g) du procédé comprend en outre l'actionnement de l'ouverture et/ou de la fermeture de la vanne de dérivation.

10. Programme informatique configuré pour implémenter le procédé selon l'une quelconque des revendications 1 à 9 lorsqu'il est exécuté sur le dispositif de commande des moyens de commande (9) du système tel que défini dans l'une quelconque des revendications 1 à 9.
